(19) **Europäisches Patentamt**
**European Patent Office**
**Office européen des brevets**

(11) **EP 2 007 462 B1**

(12) **EUROPEAN PATENT SPECIFICATION**

(45) Date of publication and mention
of the grant of the patent:
**14.06.2017 Bulletin 2017/24**

(51) Int Cl.:
***A61M 21/00*** *(2006.01)* ***G09G 5/02*** *(2006.01)*
***A61N 1/06*** *(2006.01)*

(21) Application number: **07735364.7**

(22) Date of filing: **03.04.2007**

(86) International application number:
**PCT/IB2007/051180**

(87) International publication number:
**WO 2007/116341 (18.10.2007 Gazette 2007/42)**

(54) **CONTROLLING A PHOTO-BIOLOGICAL EFFECT WITH LIGHT**

KONTROLLIEREN EINES FOTOBIOLOGISCHEN EFFEKTS MIT LICHT

REGULATION LUMINEUSE D'UN EFFET PHOTO-BIOLOGIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.04.2006 EP 06112471**

(43) Date of publication of application:
**31.12.2008 Bulletin 2009/01**

(73) Proprietor: **Philips Lighting Holding B.V.**
**5656 AE Eindhoven (NL)**

(72) Inventors:
• **DE BOER, Dirk, K., G.**
**NL-5656 AA Eindhoven (NL)**

• **SCHLANGEN, Lucas, J., M.**
**NL-5656 AA Eindhoven (NL)**
• **DE VAAN, Adrianus, J., S., M.**
**NL-5656 AA Eindhoven (NL)**

(74) Representative: **Verweij, Petronella Daniëlle et al**
**Philips Lighting B.V.**
**Philips Lighting Intellectual Property**
**High Tech Campus 45**
**5656 AE Eindhoven (NL)**

(56) References cited:
**EP-A- 1 596 357       EP-A1- 1 204 087**
**DE-A1- 4 108 328      JP-A- 2005 063 687**
**US-A1- 2004 093 045   US-A1- 2005 185 399**
**US-A1- 2006 152 525**

EP 2 007 462 B1

**Description**

FIELD OF THE INVENTION

The invention relates to a device for generating at least blue light, a display device comprising pixels for generating the at least blue light, a backlight unit for a display device, and a method of generating at least blue light.

BACKGROUND OF THE INVENTION

JP2005-063687 discloses that a living body has a timer which defines the circadian rhythm of the body. Sleepiness, alertness and temperature change according to the circadian rhythm. The biorhythm is controlled by the amount of melatonin secretion. It was found that light influences the melatonin secretion. The secretion of melatonin is maximally suppressed by light having a wavelength of 470 nm. JP2005-063687 further discloses a light-emitting device and display device exerting a biological rhythm control by emitting blue light with a wavelength of 445 to 480 nm. The light-emitting device has red LEDs, green LEDs, first blue LEDs, and second blue LEDs. The first blue LEDs emit light with a peak at 470 nm, the second blue LEDs emit light with a peak at a shorter wavelength than the first blue LEDs. The melatonin restriction effect is controlled by selecting between the first blue LEDs and the second blue LEDs.
It is a drawback of this light emitting device that the color and/or intensity of the light varies when the melatonin suppression effect is changed.
US2005/0185399 discloses a device according to the preamble of claim 1.

SUMMARY OF THE INVENTION

It is an object of the invention to provide a device for varying light to obtain a different effect on the melatonin suppression but substantially the same color and intensity.
A first aspect of the invention provides a device for generating at least blue light as claimed in claim 1. A second aspect of the invention provides a display device comprising pixels as claimed in claim 10. A third aspect of the invention provides a backlight unit for a display device as claimed in claim 18. A fourth aspect of the invention provides a method of generating at least blue light as claimed in claim 19. Advantageous embodiments are defined in the dependent claims.
A device in accordance with the first aspect of the invention generates at least blue light and has a control circuit which varies the spectrum of the blue light dependent on a control signal to control a photo-biological effect of a vertebrate. The photo-biological effect may be a melatonin suppression effect and/or a biological stimulating/alerting effect on subjects without any measurable effect on melatonin levels. The spectrum of the blue light can be varied by generating first blue light with a first predominant wavelength having a first photo-biological effect, and/or by generating a second blue light with both a second predominant wavelength, being shorter than the first predominant wavelength, and a third predominant wavelength, being longer than the first predominant wavelength. The second blue light has a second photo-biological effect smaller than the first photo-biological effect, while the first blue light and the second blue light have substantially identical colors and perceived intensities. Switching between the first and the second blue light may be instantaneous, but is preferably a slow transition which may take hours, such that the light slowly changes from the first to the second photo-biological effect, or the other way around.
The first blue light has a predominant first wavelength in between the predominant second and third wavelengths of the second blue light. By using this single blue light source or the two blue light sources in combination with each other, it is possible to obtain substantially the same color and intensity of the blue light but different photo-biological effects.
It is especially important that the color and intensity of the blue light do not change substantially in applications where the blue light is used as one of the primaries of a color display. This allows to produce the same visual image but with different photo-biological effects. By substantially the same color and intensity is meant that the viewer does not observe a change of the color and/or intensity irrespective of whether the first or the second blue light is used for the blue primary. In a backlight unit, the blue light may be produced by one or more lamps or LEDs. In a CRT or PDP, the blue light may be produced by phosphor dots or stripes. The skilled person readily understands that for creating a substantially identical color and intensity, there are many possibilities to select the two predominant wavelengths of the second blue light and the intensity thereof. From the fact that a different photo-biological effect has to be reached it is clear that the skilled person, knowing the photo-biological curve as a function of the wavelength, has many possibilities to select the wavelengths of the first and the second blue light and their associated intensities.
In an embodiment for melatonin suppression, the predominant first wavelength of the first blue light is selected in a range of 460 to 480 nm, thus near to the maximum of the melatonin suppression curve, which occurs at about 470 nm. Preferably, the predominant first wavelength is selected to coincide with this maximum. The second and third wavelengths of the second blue light are now selected on either side of the predominant first wavelength and thus at wavelengths at which the melatonin suppression is lower than maximum. Preferably, the second predominant wavelength is selected in a range of 430 to 450 nm, and the third predominant wavelength is selected in a range of 480 to 500 nm. These ranges are preferred because they have a different melatonin suppression effect and are within the non-zero part of the visual eye sensitivity curve.

In an embodiment, a first light source generates the first blue light, a second light source generates the light with the second predominant wavelength, and a third light source generates the light with the third predominant wavelength. Controlling three separate light sources is easier than changing the spectrum of one light source. Preferably, the light sources are LEDs. Alternatively, the light sources may be formed by suitably selected phosphors which are hit by electrons, such as in a CRT or PDP display apparatus.

In an embodiment, the control signal which controls whether the first blue light or the second blue light is generated is received by a wired or wireless link, for example via the Internet or telephone. This allows controlling the amount of melatonin suppression from a central point. The control signal may be linked to the time to synchronize the amount of melatonin suppression with the real day/night cycle. Alternatively, the amount of melatonin suppression may be controlled in accordance with an artificial day/night cycle for people who, for example, have to work in night shifts.

Alternatively, a light sensor maybe used to control the amount of melatonin suppression. Preferably, this light sensor is positioned to receive outside light. Even if a person is working in an environment in which no or only a low amount of daylight enters, it is possible to synchronize the selection of the spectral composition of the blue light such that the melatonin suppression is linked to the real day/night cycle.

In an embodiment, the display device further comprises sensing means for generating the control signal in dependence on biofeedback from a user. Now, the actual biological state of the user is used to control the photo-biological effect. The sensing means may comprise at least one out of: a skin/rectal temperature sensor, eye blinking sensor, eye movement sensor, skin conductance sensor, or a user activity detector. Each one of these sensors senses a particular issue of the biological state of the user, and may be used separately or in any combination to control the photo-biological effect in a desired manner. The user-activity detector may be constructed for sensing a number of keystrokes per minute, or the intensity of the use of a mouse.

In one application, the first, second and third light sources, which all generate blue light, are incorporated in the pixels of a display apparatus. Preferably, these pixels further also have a red and a green light source such that the pixels are able to produce white light. In accordance with the invention, the blue primary of the display can have different spectral compositions such that different melatonin suppression effects are obtained but still substantially the same blue color and intensity is achieved. Consequently, the white color of the display is substantially independent of the actual melatonin suppression effect selected. Preferably, the light sources are narrow band or monochromatic, such as LEDs or lasers.

In another application, in a backlight unit for illuminating a display, the first, second and third light sources are combined with red and green light sources to obtain white light the color point of which is substantially independent of the blue light sources that are activated.

These and other aspects of the invention are apparent from and will be elucidated with reference to the embodiments described hereinafter.

BRIEF DESCRIPTION OF THE DRAWINGS

In the drawings:

Fig. 1 shows the human eye sensitivity curves for the red, green and blue cones,
Fig. 2 shows x, y, z curves according to the CIE 1931 standard observer,
Fig. 3 shows the effect of a light source with a wavelength corresponding to the peak of the melatonin sensitivity curve,
Fig. 4 shows the combined effect of two light sources with wavelengths selected around the wavelength corresponding to the peak of the melatonin sensitivity curve,
Fig. 5 shows an embodiment of a display apparatus comprising an LCD panel and a backlight unit with LED light sources in accordance with the present invention,
Fig. 6 shows a CRT with phosphor light sources, and
Fig. 7 shows the CIE1931 chromaticity diagram.

It should be noted that items which have the same reference numbers in different Figures, have the same structural features and the same functions, or are the same signals. In cases where the function and/or structure of such an item has been explained, there is no necessity for repeated explanation thereof in the detailed description.

DETAILED DESCRIPTION

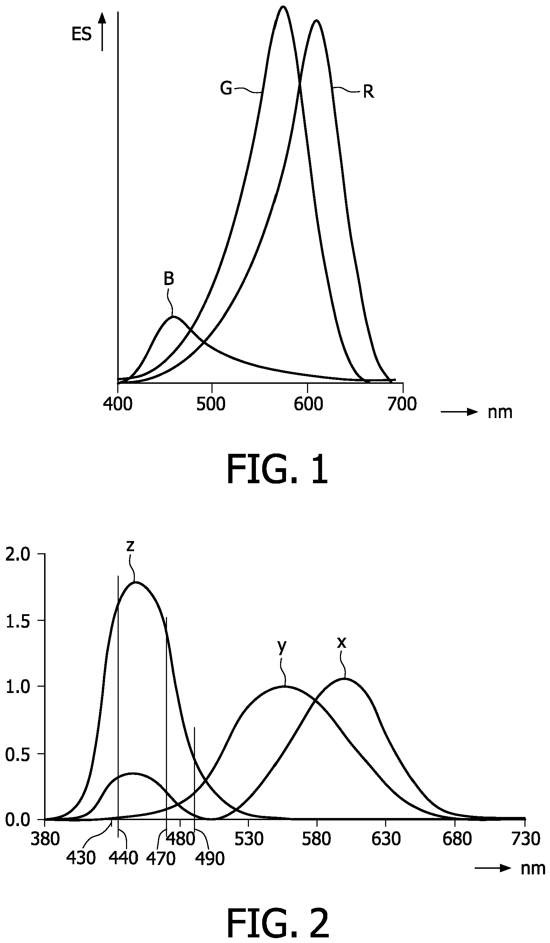

Fig. 1 shows the human eye sensitivity curves for the red, green and blue cones. The wavelength of the light is indicated along the horizontal axis in nm, the human eye sensitivity ES is indicated along the vertical axis. The human retina has three kinds of cones: cones which are sensitive to red light and which are referred to as red cones, cones which are sensitive to green light and which are referred to as green cones, and cones which are sensitive to blue light and which are referred to as blue cones. The response of the red cones as a function of the wavelength of the incident light is shown by the curve indicated by R. The response of the green cones as a function of the wavelength of the incident light is shown by the curve indicated by G. The response of the blue cones as a function of the wavelength of the incident light is shown by the curve indicated by B. The red cones have maximum sensitivity at 580 nm, the green cones have maximum sensitivity at 545 nm, and the blue cones have maximum sensitivity at 440 nm.

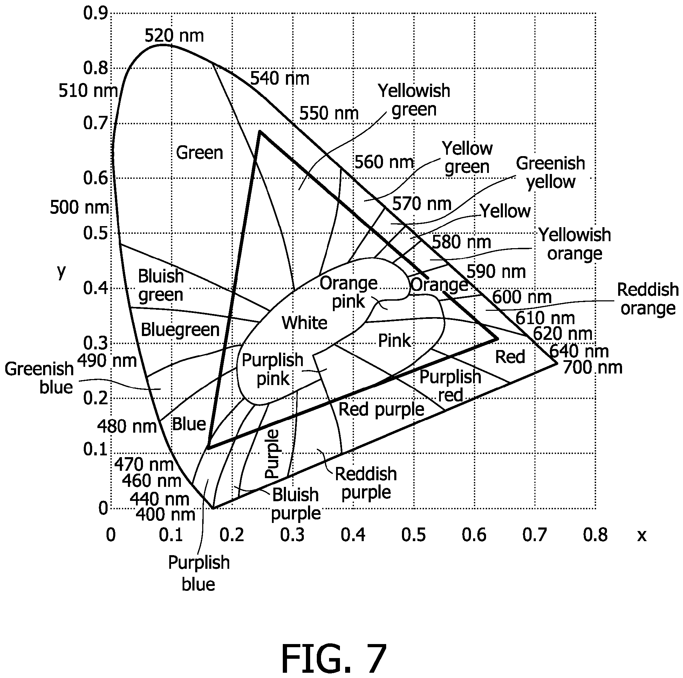

Fig. 2 shows x, y, z Standard Colorimetric Observer XYZ functions according to the CIE 1931 standard observer. Fig. 2 shows the color-matching functions as standardized by the CIE (ISO/CIE 10527: http://www.cie.co.at/publ/abst/10527.html; expected to be replaced soon by CIE Draft Standard DS 014-1.2/E:2004: http://www.cie.co.at/publ/abst/ds014_1.pdf). The curves of Figure 2 are used to calculate the x, y value of a light spectrum to locate a particular color within the CIE1931 chromaticity diagram (see Figure 7), using the formulas:

$$X = \int_0^\infty I(\lambda)\, \overline{x}(\lambda)\, d\lambda$$

$$Y = \int_0^\infty I(\lambda)\, \overline{y}(\lambda)\, d\lambda$$

$$Z = \int_0^\infty I(\lambda)\, \overline{z}(\lambda)\, d\lambda$$

where $I(\lambda)$ is the spectral power distribution (Watt/nm) of the light, $\lambda$ is the wavelength of the light, and $x(\lambda)$, $y(\lambda)$ and $z(\lambda)$ are the CIE 1931 Standard Colorimetric Observer XYZ functions. The CIE1931 chromaticity co-ordinates (x, y values) are calculated as:

$$x = \frac{X}{X+Y+Z} \qquad \text{and} \qquad y = \frac{Y}{X+Y+Z}$$

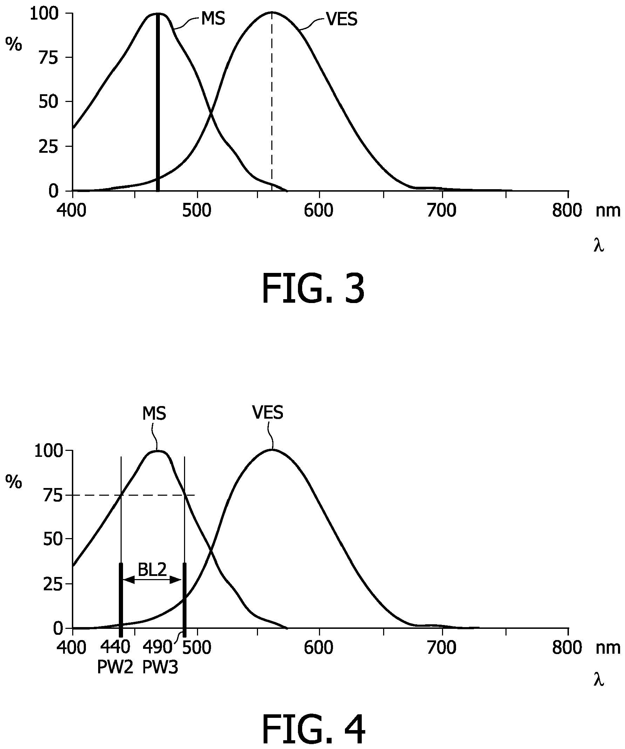

Fig. 3 shows the effect of a light source with a wavelength corresponding to the peak of the melatonin sensitivity curve. The melatonin suppression curve MS shows that the melatonin suppression effect has a maximum at 470 nm. The curve VES shows that the visual eye sensitivity has a maximum at about 560 nm. The visual eye sensitivity curve is determined by the three response curves R, G and B shown in Fig. 1.

By way of example, it is assumed that the blue light is generated with an intensity of 1 W/nm at a wavelength of 470 nm. Thus, this blue light has a wavelength which coincides with the maximum of the melatonin suppression effect, and has a normalized melatonin suppressing stimulus of 100%. The visual CIE1931 properties of this blue light are defined by:

$$x = 0.1954 / (0.1954 + 0.910 + 1.2876) = 0.124$$

$$y = 0.0910 / (0.1954 + 0.910 + 1.2876) = 0.058$$

$$Y = 683 * 0.0910 = 62 \text{ lumen.}$$

The CIE 1931 Standard Colorimetric Observer XYZ is a model to describe the color appearance of light as seen by the average human eye. Spots of light having the same x, y coordinates in the CIE 1931 chromaticity diagram and the same Y value are observed as identically colored spots of light, independent of the spectral composition of these spots of light.

Fig. 4 shows the combined effect of two light sources with wavelengths selected around the wavelength corresponding to the peak of the melatonin suppression curve. The same melatonin suppression curve MS and visual eye sensitivity curve VES as in Fig. 3 are shown. The first one of the light sources generates blue light having a wavelength of 440 nm and an intensity of 0.361 W/nm, the second one of the light sources generates blue light having a wavelength of 490 nm and an intensity of 0.397 W/nm.

The resulting combined blue light has a total normalized melatonin suppressing stimulus of 57%, and the visual properties are defined by

$$x = 0.132$$

$$y = 0.087$$

$$Y = 683 * 0.0910 = 62 \text{ lumen}$$

The melatonin suppressing stimulus is calculated by adding the contribution of the blue light at 440 nm, which is 0.361 * 0.75, to the contribution of the blue light at 490 nm, which is 0.397 * 0.77.

The values x, y, Y are calculated by adding together the contributions of the blue light at 440 nm and at 490 nm:

$$x = (0.3483 * 0.361 + 0.0320 * 0.397) / N$$

$$y = (0.0230 * 0.361 + 0.2080 * 0.397) / N$$

$$Y = 683 * y * N$$

$$N = (0.3483 * 0.361 + 0.0320 * 0.397) + (0.0230 * 0.361 + 0.2080 * 0.397) +$$
$$(1.7471 * 0.361 + 0.4652 * 0.397)$$

From Figs. 3 and 4 and the corresponding calculations it follows that a switch over between generating the blue light by a single source with a wavelength of 470 nm and generating the blue light by two sources with a wavelength of 440 nm and 490 nm, respectively, changes the melatonin suppressing stimulus from 100% to 57%, while the visual stimulus has substantially the same color (x, y changes from 0.124, 0.058 to 0.132, 0.087) and a substantially identical luminance (Y = 62 lumen in both cases).

Thus, in the terminology used in the claims, the combination of the light with the predetermined second wavelength (440 nm in this embodiment) and the predetermined third wavelength (490 nm in this embodiment) has substantially the same color and intensity as the light with the predetermined first wavelength (470 nm in this embodiment). Preferably, the first wavelength is selected at, or around, the maximum of the melatonin suppression curve MS. For example, the first wavelength is selected in the range from 460 to 480 nm. The second wavelength is selected to be shorter than the first wavelength. Preferably, the second wavelength is selected in the range from 430 to 450 nm. The second wavelength should be selected within the non-zero part of the visual eye sensitivity curve VES. The third wavelength is selected to be longer than the first wavelength. Preferably, the third wavelength is selected in the range from 480 to 500 nm. The difference between the second and third wavelengths with respect to the first wavelength is determined by the desired difference in melatonin suppression effect. The intensity of the light with the second and third wavelengths is selected such that the combined intensity is substantially identical to the intensity of the light with the first wavelength. Further, the intensity of the light with the second and third wavelengths has to be selected such that the color of the light of the first wavelength and the color of the combined light of the second and the third wavelength are substantially the same. Small differences in color and/or luminance are allowable. Preferably, the observer does not see any noticeable differences between the different lights. To further boost the melatonin suppression effect without influencing the visual appearance, it is possible to add a light source with such a short wavelength that it is invisible but still within the non-zero part of the melatonin suppression curve MS.

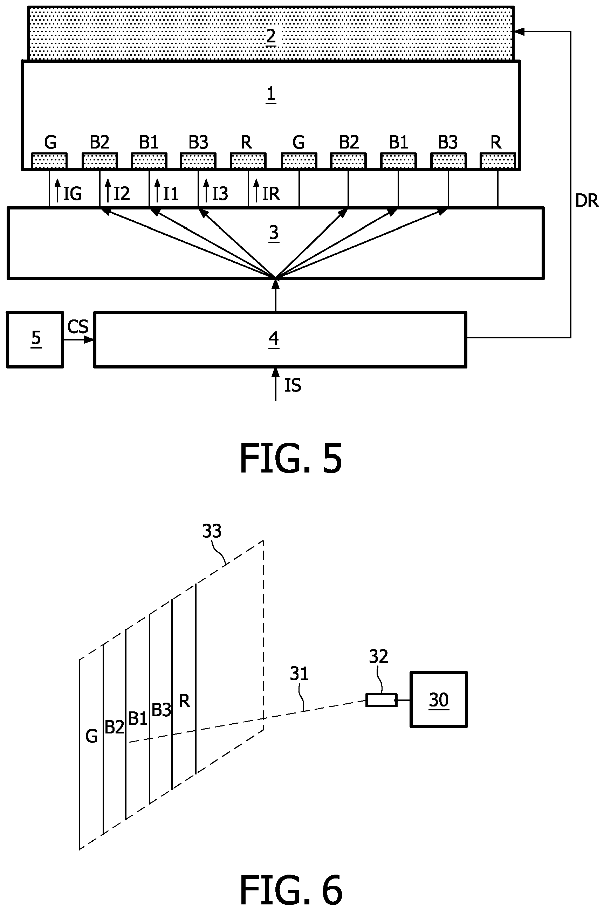

Fig. 5 shows an embodiment of a display apparatus comprising an LCD panel and a backlight unit with LED light sources in accordance with the present invention. The display apparatus comprises the backlight unit 1 which illuminates the LCD panel 2. The backlight unit 1 comprises an array of LEDs. The green LEDs G emit green light, the red LEDs R emit red light, the blue LEDs B1 emit blue light at a wavelength of predominantly 470 nm, the blue LEDs B2 emit blue light at a wavelength of predominantly 440 nm, and the blue LEDs B3 emit blue light at a wavelength of predominantly 490 nm. By a wavelength predominantly at a particular nm is meant that the LED emits light at only this wavelength, or in a small range around this wavelength, or that the intensity of the light has a maximum at this particular wavelength. As shown in Fig. 5, preferably, to optimize the spatial resolution, the blue LEDs B1 are positioned in between the blue LEDs B2 and B3.

A driver 3 supplies currents IG, IR, I1, I2, I3 to the green LEDs G, the red LEDs R, the blue LEDs B1, the blue LEDs B2, and the blue LEDs B3, respectively. A controller 4 controls the driver 3 to supply the currents IG, IR, I1, I2, I3 corresponding to a desired melatonin suppression effect, color and intensity.

The controller receives a control signal CS from a control signal generating circuit 5 which, for example, may comprise a time generator, a light sensitive element, or a trigger circuit.

The time generator generates the control signal CS for switching at predetermined switching instants between generating the blue light either by the LEDs B1 or by the combination of the LEDs B2 and B3. These switching instants may be synchronized with a real or artificial day/night cycle. Artificial day/night cycling may be interesting, for example, for people who have to work at night or who live in a situation where they are not exposed to the real day/night cycling. It is not required that, at the switching instants, the current through the LEDs B1 is switched off completely; it is possible to gradually dim the LEDs B1 while the brightness of the LEDs B2 and B3 is gradually increased. The same is true for a switch over from the LEDs B2 and B3 to the LEDs B1. This gradual switch over may occur within several hours.

The light sensitive element maybe used to receive real daylight and to generate a control signal CS which controls the switch over in synchronism with the outside light conditions. This is especially interesting in situations where a person does not receive sufficient daylight, or receives predominantly light emitted by the display apparatus.

The trigger circuit may be coupled, wired or wirelessly (via telephone or the Internet), with a central system, usually a server, which controls the switch over and thereby the variation of the melatonin suppressing effect.

The control signal may also depend on a biophysical input or combinations thereof, such as, for example, body temperature, eye blinking frequency, computer keyboard/mouse use (for example intensity and/or speed of movement of the mouse) to measure fatigue and to adjust the color of the light to a desired alertness/sleepiness setting.

In a display apparatus, the controller 4 further receives an image signal IS which determines the image to be displayed on the LCD panel and supplies a drive signal DR to the LCD panel to modulate the transmission of the pixels in accordance with the image signal IS. Alternatively, if the LCD panel is not present and the matrix of LEDs forms the display panel, this image signal IS controls the currents through the LEDs G, R, B1 or G, R, B2, B3 in accordance with the image signal IS such that the image is displayed. In another alternative, in a display apparatus with a LCD panel an option exists to put the LCD panel in a predetermined transmission state (preferably maximum), which is not controlled anymore by the image signal IS, so that the display apparatus can be used as a bright light generator. Software may ask the user some questions, i.e. to provide advice on, or to determine, the duration of the exposure to the light, the variation of the blue spectrum over time, and the intensity of the light. If the bright light is used to cater for a time shift of the day/night cycle, for example due to air travel, the questions may relate to the current time at the departure location and the most recent wake up time. The software may require input on the current time at the destination location.

An example of the variation of the blue spectrum is given next. In the early morning, when the person starts working, for example at 8 o'clock, the light generated has a high melatonin suppression effect which gradually decreases to a minimum just before lunch. After lunch the melatonin suppressing effect is increased steeply and then decreases slowly again until the person leaves his workspace. Optionally, just before the person goes home the light may be changed to increase the melatonin suppressing effect, stimulating the alertness of the person during travel and reducing accident risk.

Fig. 6 shows a CRT with phosphor light sources. The display screen 33 of the CRT (Cathode Ray Tube) comprises phosphor stripes. The unit 32 comprises an electron gun for generating an electron beam 31 with a controllable intensity and a deflection unit for deflecting the electron beam 31 to the desired position on the screen 33. The phosphors emit light when hit by the electron beam 31. The color of light emitted by the phosphors is indicated by G for green, R for red, B1 for the first blue color, B2 for the second blue color, and B3 for the third blue color. Instead of phosphor stripes, phosphor dots may be used. Usually, in a color CRT with three different phosphors, three electron beams, one for each color phosphor, are generated which are separately controllable. In the display in accordance with the present invention, five separately controllable electron beams may be generated, of which 3 (R, G, B1) are active when maximum melatonin suppression is required or 4 (R, G, B2, B3) are active when minimum melatonin suppression is required. During a transition phase between these two states, 5 electron beams (R, G, B1, B2, B3) are active. In a PDP (Plasma Display Panel), the electrons are generated by an ignition of plasma. The controller 30 controls the intensity of the electron beam or beams 31 and the deflection thereof, as is well known from display apparatuses which comprise a CRT.

Fig. 7 shows the CIE1931 chromaticity diagram. In this well known diagram the X is depicted along the horizontal axis and the Y is depicted along the vertical axis. Because the Figure is in black and white, areas are indicated by their color's name. As is well known, although specific areas of colors are shown, these colors gradually change.

It should be noted that the above-mentioned embodiments illustrate rather than limit the invention, and that those skilled in the art will be able to design many alternative embodiments without departing from the scope of the appended claims.

For example, the LEDs of the backlight unit 1 maybe OLEDs, lasers, gas discharge lamps, or fluorescent tubes, or any combination thereof. The discharge lamps may comprise different light sources in the same bulb. Instead of an LCD panel 2 in front of the backlight unit 1, any other display panel with a locally controllable transmission can be used. The present invention may be implemented in all apparatuses with a display device, such as, for example, television sets, computer monitors, PDAs, mobile phones, photo and film cameras.

Alternatively, the display unit may be absent altogether and the backlight unit is a lighting unit for generating so called "bright light therapy".

It is not required that the different blue spectrums are exact metamerisms, slight deviations are allowed. If the light is used in a display it is possible to electronically correct for these deviations, for example by slightly adapting the currents through the LEDs.

Instead of a day/night synchronization of the blue spectrum, in special conditions other synchronizations are possible to control the biological rhythm. Such special conditions may be: traveling in boats, airplanes, spaceships, submarines; locations on earth near the poles where the light/dark cycles strongly change over the year; or people that work in night shifts.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. In the device claim enumerating several means, several of these means may be embodied by one and the same item of hardware. The mere fact that certain measures are recited in mutually different dependent claims does not indicate that a combination of these measures cannot be used to advantage.

**Claims**

1. A device for generating at least blue light and having a control circuit (4) for receiving a control signal (CS) defining a variation of a spectrum of the blue light to control a photo-biological effect of a vertebrate by generating first blue light (BL1) with a first predominant wavelength (PW1) having a first photo-biological effect, and/or second blue light (BL2) with both a second predominant wavelength (PW2), being shorter than the first predominant wavelength, and a third predominant wavelength (PW3), being longer than the first predominant wavelength, wherein the second blue light (BL2) has a second photo-biological effect smaller than the first photo-biological effect, **characterized in that** the first blue light (BL1) and the second blue light (BL2) have substantially identical colors and intensities.

2. A device as claimed in claim 1, wherein the first predominant wavelength (PW1) is selected in a range of 460 to 480 nm, the second predominant wavelength (PW2) is selected in a range of 430 to 450 nm, and the third predominant wavelength (PW3) is selected in a range of 480 to 500 nm.

3. A device as claimed in claim 1, further comprising a first light source (B1) for generating the first blue light (BL 1), a second light source (B2) for generating blue light with the second predominant wavelength (PW2), and a third light source (B3) for generating blue light with the third predominant wavelength (PW3).

4. A device as claimed in claim 3, wherein the first light source (B1) comprises a first LED, the second light source (B2) comprises a second LED, and the third light source (B3) comprises a third LED, and the device further comprises a drive circuit (3) for generating a first current (I1) through the first LED, a second current (I2) through the second LED, and a third current (I3) through the third LED, and the control circuit (4) is constructed for controlling the drive circuit (3) to generate the first current (I1), the second current (I2), and the third current (I3) to obtain light having a desired color, intensity, and a photo-biological effect.

5. A device as claimed in claim 3, wherein the first light source (B1) is a first type of phosphor, the second light source (B2) is a second type of phosphor, and the third light source (B3) is a third type of phosphor, and the device further comprises a drive circuit (30) for deflecting an electron beam (31) to either hit the first type of phosphor or both the second type of phosphor and the third type of phosphor.

6. A device as claimed in any one of the preceding claims, wherein the control signal (CS) is a trigger signal obtained by a wired or wireless link.

7. A device as claimed in any one of the preceding claims, further comprising a time generating circuit (5) for generating the control signal (CS) having a cyclical behaviour to control the photo-biological effect cyclically.

8. A device as claimed in claim 7, wherein the time generating circuit (5) is constructed for generating the control signal (CS) synchronized with the day/night cycle.

9. A device as claimed in claim 6, further comprising a light sensitive element (5) for generating the trigger signal (CS) in response to an amount of light impinging on the light sensitive element.

10. A display device **characterized in that** it comprises pixels, each comprising the first light source (B1), the second light source (B2) and the third light source (B3), as claimed in claim 3 and a control circuit (4) as claimed in claim 1.

11. A display device as claimed in claim 10, wherein the pixels each further comprise a fourth light source (G) and a fifth light source (R) for enabling the pixels to emit white light.

12. A display device as claimed in claim 11, wherein the fourth light source (G) emits green light and the fifth light source (R) emits red light.

13. A display device as claimed in claim 11 or 12, wherein the first light source (B1) comprises a first LED, the second light source (B2) comprises a second LED, the third light source (B3) comprises a third LED, the fourth light source (G) comprises a fourth LED, and the fifth light source (R) comprises a fifth LED, and the display device further comprises a drive circuit (3), and the control circuit (4) is constructed for receiving the control signal (CS) and an image signal (IS) to control the drive circuit (3) to generate a first current (I1) through the first LED, a second current (I2) through the second LED, and a third current (I3) through the third LED, a fourth current (IG) through the fourth LED, and a fifth current (IR) through the fifth LED, to obtain light having a desired color and intensity in accordance with the image signal (IS), and a photo-biological effect.

14. A display device as claimed in claim 10, further comprising sensing means for generating the control signal depending on biofeedback from a user.

15. A display device as claimed in claim 14, wherein the sensing means comprises at least one out of: a skin/rectal temperature sensor, eye blinking sensor, eye movement sensor, skin conductance sensor, or a user-activity detector.

16. A display device as claimed in claim 15, wherein the user-activity detector is constructed for sensing a number of keystrokes per minute, or the intensity of a mouse use.

17. A display device as claimed in any of claims 10 to 16 wherein the photo-biological effect is a melatonin suppression effect or an alertness level.

18. A backlight unit for a display device **characterized in that** it comprises the first light source (B1), the second light source (B2) and the third light source (B3), as claimed in claim 3 and a control circuit (4) as claimed in claim 1.

19. A method of generating at least blue light in response to a control signal (CS) defining a variation of a spectrum of the blue light to control a photo-biological effect of a vertebrate by generating first blue light (BL1) with a first predominant wavelength (PW1) having a first melatonin suppression effect, and/or second blue light (BL2) with both a second predominant wavelength (PW2), being shorter than the first predominant wavelength, and a third predominant wavelength (PW3), being longer than the first predominant wavelength, wherein the second blue light (BL2) has a second melatonin suppression effect smaller than the first melatonin suppression effect, **characterized in that** the first blue light (BL1) and the second blue light (BL2) have substantially identical colors and intensities.

**Patentansprüche**

1. Vorrichtung zur Erzeugung von zumindest blauem Licht, die einen Steuerkreis (4) zum Empfang eines Steuersignals (CS) aufweist, das eine Variation eines Spektrums des blauen Lichts definiert, um einen fotobiologischen Effekt eines Vertebrats durch Erzeugen von erstem blauem Licht (BL1) mit einer ersten predominanten Wellenlänge (PW1) mit einem ersten fotobiologischen Effekt und/oder zweitem blauem Licht (BL2) mit sowohl einer zweiten predominanten Wellenlänge (PW2), die kürzer als die erste predominante Wellenlänge ist, als auch einer dritten predominanten Wellenlänge (PW3), die länger als die erste predominante Wellenlänge ist, zu steuern, wobei das zweite blaue Licht (BL2) einen zweiten fotobiologischen Effekt aufweist, der kleiner als der erste fotobiologische Effekt ist, **dadurch gekennzeichnet, dass** das erste blaue Licht (BL1) und das zweite blaue Licht (BL2) im Wesentlichen identische Farben und Intensitäten aufweisen.

2. Vorrichtung nach Anspruch 1, wobei die erste predominante Wellenlänge (PW1) in einem Bereich von 460 bis 480 nm ausgewählt wird, die zweite predominante Wellenlänge (PW2) in einem Bereich von 430 bis 450 nm ausgewählt wird und die dritte predominante Wellenlänge (PW3) in einem Bereich von 480 bis 500 nm ausgewählt wird.

3. Vorrichtung nach Anspruch 1, die weiterhin eine erste Lichtquelle (B1) zur Erzeugung des ersten blauen Lichts (BL1), eine zweite Lichtquelle (B2) zur Erzeugung von blauem Licht mit der zweiten predominanten Wellenlänge (PW2) sowie eine dritte Lichtquelle (B3) zur Erzeugung von blauem Licht mit der dritten predominanten Wellenlänge (PW3) umfasst.

4. Vorrichtung nach Anspruch 3, wobei die erste Lichtquelle (B1) eine erste LED umfasst, die zweite Lichtquelle (B2) eine zweite LED umfasst und die dritte Lichtquelle (B3) eine dritte LED umfasst, und wobei die Vorrichtung weiterhin eine Ansteuerungsschaltung (3) umfasst, um einen ersten Strom (I1) durch die erste LED, einen zweiten Strom (I2) durch die zweite LED sowie einen dritten Strom (I3) durch die dritte LED zu erzeugen, und wobei der Steuerkreis (4) so eingerichtet ist, dass er die Ansteuerungsschaltung (3) so steuert, dass der erste Strom (I1), der zweite Strom (I2) und der dritte Strom (I3) erzeugt werden, um Licht zu erhalten, das eine gewünschte Farbe, Intensität sowie einen fotobiologischen Effekt aufweist.

5. Vorrichtung nach Anspruch 3, wobei es sich bei der ersten Lichtquelle (B1) um einen ersten Leuchtstofftyp, bei der zweiten Lichtquelle (B2) um einen zweiten Leuchtstofftyp und bei der dritten Lichtquelle (B3) um einen dritten Leuchtstofftyp handelt, und wobei die Vorrichtung weiterhin eine Ansteuerungsschaltung (30) umfasst, um einen Elektronenstrahl (31) so abzulenken, dass dieser entweder auf den ersten Leuchtstofftyp oder auf sowohl den zweiten Leuchtstofftyp als auch den dritten Leuchtstofftyp auftrifft.

6. Vorrichtung nach einem der vorangegangenen Ansprüche, wobei das Steuersignal (CS) ein durch eine drahtgebundene oder drahtlose Verbindung erhaltenes Auslösesignal ist.

7. Vorrichtung nach einem der vorangegangenen Ansprüche, die weiterhin eine Zeiterzeugungsschaltung (5) zur Erzeugung des Steuersignals (CS) umfasst, das ein zyklisches Verhalten aufweist, um den fotobiologischen Effekt zyklisch zu steuern.

8. Vorrichtung nach Anspruch 7, wobei die Zeiterzeugungsschaltung (5) so eingerichtet ist, dass sie das mit dem Tages-/Nachtzyklus synchronisierte Steuersignal (CS) erzeugt.

9. Vorrichtung nach Anspruch 6, die weiterhin ein lichtempfindliches Element (5) umfasst, um in Reaktion auf eine auf das lichtempfindliche Element auftreffende Lichtmenge das Auslösesignal (CS) zu erzeugen.

10. Anzeigevorrichtung, **dadurch gekennzeichnet, dass** diese Pixel umfasst, die jeweils die erste Lichtquelle (B1), die zweite Lichtquelle (B2) und die dritte Lichtquelle (B3) nach Anspruch 3 sowie einen Steuerkreis (4) nach Anspruch 1 umfassen.

11. Anzeigevorrichtung nach Anspruch 10, wobei die Pixel jeweils weiterhin eine vierte Lichtquelle (G) und eine fünfte Lichtquelle (R) umfassen, damit die Pixel weißes Licht emittieren können.

12. Anzeigevorrichtung nach Anspruch 11, wobei die vierte Lichtquelle (G) grünes Licht emittiert und die fünfte Lichtquelle (R) rotes Licht emittiert.

13. Anzeigevorrichtung nach Anspruch 11 oder 12, wobei die erste Lichtquelle (B1) eine erste LED umfasst, die zweite Lichtquelle (B2) eine zweite LED umfasst, die dritte Lichtquelle (B3) eine dritte LED umfasst, die vierte Lichtquelle (G) eine vierte LED umfasst und die fünfte Lichtquelle (R) eine fünfte LED umfasst, und wobei die Anzeigevorrichtung weiterhin eine Ansteuerungsschaltung (3) umfasst, und der Steuerkreis (4) so eingerichtet ist, dass er das Steuersignal (CS) und ein Bildsignal (IS) empfängt, um die Ansteuerungsschaltung (3) so zu steuern, dass diese einen ersten Strom (I1) durch die erste LED, einen zweiten Strom (I2) durch die zweite LED und einen dritten Strom (I3) durch die dritte LED, einen vierten Strom (IG) durch die vierte LED sowie einen fünften Strom (IR) durch die fünfte LED erzeugt, um Licht zu erhalten, das eine gewünschte Farbe und Intensität entsprechend dem Bildsignal (IS) sowie einen fotobiologischen Effekt aufweist.

14. Anzeigevorrichtung nach Anspruch 10, die weiterhin Sensormittel umfast, um in Abhängigkeit eines Biofeedbacks von einem Benutzer das Steuersignal zu erzeugen.

15. Anzeigevorrichtung nach Anspruch 14, wobei das Sensormittel mindestens einen der folgenden umfasst: einen Haut-/Rektaltemperatursensor, einen Sensor zur Erfassung des Blinzelns eines Auges, einen Augenbewegungssensor, einen Hautleitwertsensor oder einen Benutzeraktivitätsdetektor.

16. Anzeigevorrichtung nach Anspruch 15, wobei der Benutzeraktivitätsdetektor so eingerichtet ist, dass er eine Anzahl Tastenanschläge pro Minute oder die Intensität einer Mausbenutzung erfasst.

17. Anzeigevorrichtung nach einem der Ansprüche 10 bis 16, wobei der fotobiologische Effekt ein Melatonin-Unterdrückungseffekt oder ein Wachheitsgrad ist.

18. Hintergrundbeleuchtungseinheit für eine Anzeigevorrichtung, **dadurch gekennzeichnet, dass** diese die erste Lichtquelle (B1), die zweite Lichtquelle (B2) und die dritte Lichtquelle (B3) nach Anspruch 3 sowie einen Steuerkreis (4) nach Anspruch 1 umfasst.

19. Verfahren zur Erzeugung von zumindest blauem Licht in Reaktion auf ein Steuersignal (CS), das eine Variation eines Spektrums des blauen Lichts definiert, um einen fotobiologischen Effekt eines Vertebrats durch Erzeugen von erstem blauem Licht (BL1) mit einer ersten predominanten Wellenlänge (PW1) mit einem ersten Melatonin-Unterdrückungseffekt und/oder zweitem blauem Licht (BL2) mit sowohl einer zweiten predominanten Wellenlänge (PW2), die kürzer als die erste predominante Wellenlänge ist, als auch einer dritten predominanten Wellenlänge (PW3), die länger als die erste predominante Wellenlänge ist, zu steuern, wobei das zweite blaue Licht (BL2) einen zweiten Melatonin-Unterdrückungseffekt aufweist, der kleiner als der erste Melatonin-Unterdrückungseffekt ist, **dadurch gekennzeichnet, dass** das erste blaue Licht (BL1) und das zweite blaue Licht (BL2) im Wesentlichen identische Farben und Intensitäten aufweisen.

**Revendications**

1. Dispositif pour générer au moins une lumière bleue et ayant un circuit de contrôle (4) pour recevoir un signal de contrôle (CS) définissant une variation de spectre de la lumière bleue pour contrôler un effet photo-biologique d'un vertébré en générant une première lumière bleue (BL1) avec une première longueur d'onde prédominante (PW1) ayant un premier effet photobiologique, et/ou une deuxième lumière bleue (BL2) avec à la fois une deuxième longueur d'onde prédominante (PW2), qui est plus courte que la première longueur d'ondes prédominante, et une troisième longueur d'onde prédominante (PW3) qui est plus longue que la première longueur d'onde prédominante, dans lequel la deuxième lumière bleue (BL2) a un deuxième effet photobiologique plus réduit que le premier effet photo-biologique,
**caractérisé en ce que** la première lumière bleue (BL1) et la deuxième lumière bleue (BL2) ont sensiblement des couleurs et des intensités identiques.

2. Dispositif selon la revendication 1, dans lequel la première longueur d'onde prédominante (PW1) est choisie dans une plage de 460 à 480 nm, la deuxième longueur d'onde prédominante (PW2) est choisie dans une plage de 430 à 450 nm, et la troisième longueur d'onde prédominante (PW3) est choisie dans une plage de 480 à 500 nm.

3. Dispositif selon la revendication 1, comprenant en outre une première source de lumière (B1) pour générer la première lumière bleue (BL1), une deuxième source de lumière (B2) pour générer une lumière bleue avec la deuxième longueur d'onde prédominante (PW2) et une troisième source de lumière (B3) pour générer une lumière bleue avec la troisième longueur d'onde prédominante (PW3).

4. Dispositif selon la revendication 3, dans lequel la première source de lumière (B1) comprend une première LED, la deuxième source de lumière (B2) comprend une deuxième LED, et la troisième source de lumière (B3) comprend une troisième LED, et le dispositif comprend en outre un circuit de commande (3) pour générer un premier courant (I1) dans la première LED, un deuxième courant (I2) dans la deuxième LED et un troisième courant (I3) dans la troisième LED, et le circuit de contrôle (4) est construit pour contrôler le circuit de commande (3) pour générer le premier courant (I1), le deuxième courant (I2) et le troisième courant (I3) pour obtenir une lumière ayant une couleur, une intensité et un effet photobiologique souhaités.

5. Dispositif selon la revendication 3, dans lequel la première source de lumière (B1) est un premier type de phosphore, la deuxième source lumière (B2) est un deuxième type de phosphore et la troisième source de lumière (B3) est un troisième type de phosphore, et le dispositif comprend en outre un circuit de commande (30) pour dévier un faisceau d'électrons (31) de façon à heurter le premier type de phosphore ou à la fois le deuxième type de phosphore et le troisième type de phosphore.

6. Dispositif selon l'une quelconque des revendications précédentes, dans lequel le signal de contrôle (CS) est un signal déclencheur obtenu par un lien à fil ou sans fil.

7. Dispositif selon l'une quelconque des revendications précédentes, comprenant en outre un circuit de génération temporel (5) pour générer le signal de contrôle (CS) ayant un comportement cyclique pour contrôler de façon cyclique l'effet photo-biologique.

8. Dispositif selon la revendication 7, dans lequel le circuit de génération temporel (5) est construit pour générer le signal de contrôle (CS) synchronisé avec le cycle jour/nuit.

9. Dispositif selon la revendication 6, comprenant en outre un élément photosensible (5) pour générer le signal déclencheur (CS) en réponse à une quantité de lumière incidente sur l'élément photosensible.

10. Dispositif d'affichage, **caractérisé en ce qu'**il comprend des pixels, chacun comprenant la première source de lumière (B1), la deuxième source de lumière (B2) et la troisième source de lumière (B3) selon la revendication 3 et un circuit de contrôle (4) selon la revendication 1.

11. Dispositif d'affichage selon la revendication 10, dans lequel les pixels comprennent chacun une quatrième source de lumière (G) et la cinquième source de lumière (R) pour permettre aux pixels d'émettre une lumière blanche.

12. Dispositif d'affichage selon la revendication 11, dans lequel la quatrième source de lumière (G) émet une lumière verte et la cinquième source de lumière (R) émet une lumière rouge.

13. Dispositif d'affichage selon la revendication 11 ou 12, dans lequel la première source de lumière (B1) comprend une première LED, une deuxième source de lumière (B2) comprend une deuxième LED, la troisième source de lumière (B3) comprend une troisième LED, la quatrième source de lumière (G) comprend une quatrième LED, et la cinquième source de lumière (R) comprend une cinquième LED, et le dispositif d'affichage comprend en outre un circuit de commande (3) et le circuit de commande (4) est construit pour recevoir le signal de contrôle (CS) et un signal d'image (IS) pour contrôler le circuit de commande (3) pour générer un premier courant (I1) dans la première LED, un deuxième courant (I2) dans la deuxième LED, et un troisième courant (I3) dans la troisième LED, un quatrième courant (IG) dans la quatrième LED, et un cinquième courant (IR) dans la cinquième LED, pour obtenir une lumière ayant une couleur et une intensité souhaitées selon le signal d'image (IS), et un effet photobiologique.

14. Dispositif d'affichage selon la revendication 10, comprenant en outre des moyens de détection pour générer un signal de contrôle en fonction d'une rétroaction biologique d'un utilisateur.

15. Dispositif d'affichage selon la revendication 14, dans lequel le moyen de détection comprend au moins l'un parmi : un capteur de température cutanée/rectale, un capteur de clignement des yeux, un capteur de mouvement oculaire, un capteur de conduction cutanée ou un détecteur d'activité d'utilisateur.

16. Dispositif d'affichage selon la revendication 15, dans lequel le détecteur d'activité d'utilisateur est construit pour détecter un nombre de frappes par minute, ou l'intensité d'utilisation d'une souris.

17. Dispositif d'affichage selon l'une quelconque des revendications 10 à 16, dans lequel l'effet photo-biologique est un effet de suppression de la mélatonine ou un niveau de vigilance.

**18.** Unité de rétro-éclairage pour un dispositif d'affichage, **caractérisé en ce qu'**il comprend la première source de lumière (B1), la deuxième source de lumière (B2) et la troisième source de lumière (B3) selon la revendication 3 et un circuit de contrôle (4) selon la revendication 1.

**19.** Procédé de génération d'au moins une lumière bleue en réponse à un signal de contrôle (CS) définissant une variation d'un spectre de la lumière bleue pour contrôler un effet photobiologique d'un vertébré en générant une première lumière bleue (BL1) avec une première longueur d'onde prédominante (PW1) ayant un premier effet de suppression de la mélatonine et/ou une deuxième lumière bleue (BL2) avec à la fois une deuxième longueur d'onde prédominante (PW2), qui est plus courte que la première longueur d'onde prédominante, et une troisième longueur d'onde prédominante (PW3) qui est plus longue que la première longueur d'onde prédominante, dans lequel la deuxième lumière bleue (BL2) a un deuxième effet de suppression de la mélatonine inférieur au premier effet de suppression de la mélatonine,
**caractérisé en ce que** la première lumière bleue (BL1) et la deuxième lumière bleue (BL2) ont sensiblement des couleurs et des intensités identiques.

FIG. 1

FIG. 2

FIG. 3

FIG. 4

# FIG. 5

# FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005063687 A

- US 20050185399 A